# FASCICULE DE BREVET EUROPEEN

(11) **EP 0 775 315 B1**
(45) Date de publication et mention de la délivrance du brevet: **25.09.2002**
(21) Numéro de dépôt: 95927002.6
(22) Date de dépôt: 31.07.1995
(51) Int. Cl.: G01N 33/569, G01N 33/576, G01N 33/543

(54) **PROCEDE DE SEPARATION ET/OU DE DETECTION ET/OU DE QUANTIFICATION DE COMPOSE(S) INFECTIEUX ET SUPPORT POUR LA MISE EN OEUVRE DU PROCEDE**
VERFAHREN ZUR TRENNUNG UND/ODER IDENTIFIKATION UND/ODER QUANTIFIZIERUNG VON INFEKTIÖSEN MATERIALEN UND TRÄGER ZUR DURCHFÜHRUNG DIESES VERFAHRENS
METHOD FOR SEPARATING AND/OR SCREENING AND/OR QUANTIFYING ONE OR MORE INFECTIOUS COMPOUNDS AND SUPPORT FOR IMPLEMENTING SAID METHOD

(30) Priorité: 01.08.1994 FR 9409528; 01.08.1994 FR 9409529
(43) Date de publication de la demande: 28.05.1997
(73) Titulaire: Institut de Recherche pour le Développement ( IRD), 75010 Paris (FR)
(72) Inventeur: STEFAS, Elie, F-34280 La Grande-Motte (FR); RUCHETON, Marcel, F-34000 Montpellier (FR); GRAAFLAND, Hubert, F-34000 Montpellier (FR); Veas, Francisco, F-34130 Mauguio (FR)
(74) Mandataire: Peuscet, Jacques
(86) Numéro de dépôt international: FR9501032
(87) Numéro de publication internationale: WO96004559

(56) Documents cités:
- EP-A- 0 124 896
- EP-A- 0 600 088
- WO-A-91/02816
- FR-A- 2 701 319
- JOURNAL OF VIROLOGY, vol. 68, no. 4, Avril 1994 BALTIMORE, MD, pages 2415-2424, MEHDI ET AL. 'Hepatitis B virus surface antigen binds to apolipoprotein H'
- DATABASE WPI Week 9248 Derwent Publications Ltd., London, GB; AN 92-398879 & WO,A,92 19755 (YAMASA SHOYU KK) , 12 Novembre 1992
- LUBERT STRYER: "La biochimie", 1985, FLAMMARION MEDECINE-SCIENCES, FRANCE
- 'Dictionnaire Médical Masson', 1997, MASSON, PARIS
- GARNIER/DELAMARE: "Dictionnaire des termes de Médecine", 1989, MALOINE, PARIS
- GIRARD ET HIRTH: "Virologie générale et moléculaire", 1980, DOIN EDITEURS, PARIS

## Description

La présente invention concerne un procédé de séparation et/ou de détection et/ou de quantification de composé(s) infectieux dans un matériau biologique et un support pour la mise en oeuvre du procédé.

Selon la présente invention, on entend par composés infectieux, ci-après désignés de façon générique en abrégé par "CI", aussi bien des composés, en particulier protéiniques, constitutifs d'un agent infectieux, que des structures qui comprennent des composés infectieux. Ces structures sont, notamment, ou bien des agents infectieux endogènes ou exogènes, complets ou incomplets, leurs métabolites ou encore des assemblages contenant des composés constitutifs de ces agents infectieux, assemblages qui présentent certaines propriétés desdits agents infectieux, notamment la propriété d'être détectés par certains anticorps spécifiques de composés infectieux ; les CI peuvent être aussi des composés spécifiquement induits dans l'organisme par les CI précédemment définis. Parmi les CI on peut citer, par exemple, les virus, les bactéries, les champignons, les mycoplasmes et les parasites. On désignera ci-après par "CIV" un composé infectieux viral et par "CInV" un composé infectieux non-viral, c'est-à-dire de type autre que seulement viral.

On entend ici par "matériau biologique", un tissu biologique, une préparation ou un extrait issu de tissu biologique, liquide ou solide, ou un milieu naturel susceptible de contenir un composé infectieux au sens ci-dessus défini (par exemple, une eau d'écoulement). Le matériau peut aussi être un mélange d'au moins deux matériaux tels que ci-dessus définis. Un tel matériau biologique peut donc être, notamment, soit préparé à partir de tissus, d'organes, de selles ou de liquides biologiques d'un malade atteint d'une infection, par exemple virale, bactérienne, parasitaire, mycosique ou mycoplasmique, soit obtenu à partir de cultures "in vitro" ; un tel matériau biologique peut aussi être un sérum, du plasma, de l'urine, du liquide céphalorachidien, du liquide synovial, du liquide péritonéal, du liquide pleural, du liquide séminal ou du liquide ascitique.

On sait que la β2-glycoprotéine I, ci-après en abrégé "β2GPI", est une glycoprotéine plasmatique, dont la séquence a été notamment indiquée dans les articles de J. LOZIER et coll. Froc. Natl. Acad. Sci. ISA, Vol. 81, pages 3640-3644, Juillet 1984 et de T. KRISTENSEN et coll., FEBS Letters, Vol. 289, 1991, pages 183-186. La β2GPI est également appelée Apolipoprotéine H. Il a été constaté que cettre protéine présente un polymorphisme : la dénomination β2GPI sera ci-après considérée comme générique pour toutes les formes.

Dans EP-A-0 600 088, on indique que dans le syndrome antiphospholipide, il est détecté dans le sérum des anticorps anticardiolipine dirigés contre le complexe cardiolipine-β2GPI, qui apparaissent dans ce syndrome et que l'on peut doser ces anticardiolipines en faisant réagir le sérum sur le complexe cardiolipine-β2GPI. La cardiolipine est susceptible d'être associée à un composé infectieux (le tréponème pâle) mais, la cardiolipine pouvant provenir d'autres origines et, en particulier, existant en l'absence de pathologie, n'est pas un composé spécifique induit dans l'organisme par un agent infectieux et ne peut donc pas être utilisée de façon fiable pour déceler la présence dans l'organisme de cet agent infectieux.

Dans la demande internationale WO 94/18569, on a indiqué que certains composés viraux se fixaient de façon spécifique sur une forme de β2GPI, à savoir celle décrite dans la demande de brevet français 2 701 263, que cette forme de β2GPI soit à l'état pur ou dans une composition protéinique la contenant ; cette forme de β2GPI est isolée à partir du résidu fixé sur la (les) colonne(s) de chromatographie d'affinité utilisée(s) dans le procédé de purification de l'albumine du plasma sanguin décrit dans FR-A-2 690 444 ; elle a un poids moléculaire de 50 000 ± 3 000 daltons : dans le cadre de la présente demande de brevet, cette forme de β2GPI a été désignée en abrégé par "β2'GPI". On a donc proposé dans WO 94/18569 un procédé de détection et/ou de dosage de composés viraux dans lequel on fixe les composés viraux (CIV) à l'aide de β2'GPI. Dans un tel procédé, on ajoute donc la β2'GPI sur des CIV contenus dans un matériau biologique, de façon à séparer les CIV ainsi capturés pour ensuite les détecter et/ou les doser.

Il est également décrit dans Journal of Virology, 68 n° 4 Avril 1994, pages 2415-2424 que HBsAg est suceptible de former in vitro des complexes avec la β2 glycoprotéine I.

WO 92/19755 indique qu'il est possible d'obtenir un anticorps monoclonal qui réagit de façon spécifique avec la β2 glycoprotéine I humaine et permet de la doser.

De façon générale, on- appellera ici "complexe(s)", une association directe ou indirecte, entre au moins un CI et une β2GPI : ces complexes seront, de façon générale, désignés ci-après par la notation "β2GPI/CI". Le procédé décrit dans WO 94/18569 détecte et/ou dose des complexes CIV/β2'GPI entre des composés viraux CIV et la β2'GPI, à l'état pur ou dans une composition procéinique la contenant, la β2'GPI étant ajoutée au matériau biologique contenant les CIV à détecter et/ou doser.

On désignera ici par (β2GPI)n, la (ou les) forme(s) de β2GPI naturellement présente(s) dans le matériau biologique avant mise en oeuvre du procédé ci-après décrit, et non intentionnellement rajoutée en tant que telle. On désignera par (β2GPI)r la (ou les) forme(s) de r β2GPI que l'on rajoute intentionnellement pour former les complexes CI/β2GPI précédemment définis.

Selon la présente invention, on a trouvé, de façon nouvelle, qu'il était possible de séparer, détecter et/ou quantifier à partir d'un matériau biologique des complexes CI/β2GPI, autres que ceux décrits dans WO 94/18569, à savoir :
- des complexes (β2GPI)n/CI, où la partie CI peut être de type viral CIV ou non viral CInV, et la partie (β2GPI)n provient naturellement du matériau biologique étudié,
- des complexes (β2GPI)r/CInV ou la partie (β2GPI)r est rajoutée intentionnellement et préparée sous différentes formes à cet effet, pure ou en mélange, et la partie CInV provient des composés infectieux non viraux du matériau biologique étudié.

La présente invention a donc pour objet un procédé de séparation et/ou de détection et/ou de quantification dans un matériau biologique de composés infectieux (CI), en particulier protéiniques, choisis dans le groupe formé par des composés constitutifs spécifiques d'un agent infectieux, des structures qui comprennent des agents infectieux endogènes ou exogènes, complets ou incomplets, leurs métabolites, des assemblages de ces agents infectieux présentant des propriétés spécifiques des agents infectieux, des composés spécifiquement induits dans l'organisme par les agents infectieux définis ci-dessus, caractérisé par le fait que l'on sépare et/ou détecte et/ou quantifie un complexe β2GPI/CI, dans lequel β2GPI est la β2 glycoprotéine I et CI un composé infectieux, identifiable par tout moyen adapté, choisi dans le groupe formé par :
a) (β2GPI)n/CI, (β2GPI)n étant la β2 glycoprotéine I naturellement présente dans le matériau biologique, CI étant un composé viral CIV ou un composé non viral CInV,
b) (β2GPI)r/CInV, (β2GPI)r étant de la β2 glycoprotéine I rajoutée au matériau biologique et CInV étant un composé infectieux non viral.

De façon générale, pour certaines applications du procédé, la β2GPI, comme les CI, peuvent être d'origine animale ou produits par génie génétique et/ou chimique. Le procédé trouve son application tant pour l'homme que pour l'animal.

Selon l'invention, on procèdera à l'identification de la partie β2GPI des complexes par sa reconnaissance, à l'aide d'une (de) substance(s) se liant à cette partie, préférentiellement ou non, ou spécifiquement, et l'on identifiera la partie CI par tout moyen adapté.

La formation des complexes peut être directe ou indirecte, médiée ou favorisée par certains adjuvants, qui peuvent être chimiques, biochimiques ou biologiques, tels que certains lipides ou détergents, notamment des phospholipides. Les complexes peuvent se former pendant la préparation du matériau biologique et/ou durant un (des) stades du procédé.

Comme indiqué précédemment, les CI comprennent les CIV et les CInV. Parmi les CIV, on peut citer notamment ceux du groupe formé par HIV1, HIV2, HBV, HSV, des particules ou protéines d'origine virale ; parmi les CInV, on peut citer notamment ceux du groupe formé par les bactéries, les parasites, les champignons et les mycoplasmes, et plus particulièrement : pour les bactéries : Borellia ; et pour les parasites : Leishmania, infantum notamment, Toxoplasma gondii, Entamoeba histolytica.

Avantageusement, la β2GPI retenue dans le(s) complexe(s) peut, suivant les modes de mise en oeuvre du procédé, avoir été marquée ou non, ; ce marquage, préalable ou non, peut être effectué, par exemple , au moyen d'un anticorps, d'une enzyme, d'un produit radioactif, d'un produit fluorescent ou d'un agent métallique.

Selon la présente invention, on pourra réaliser un test polyspécifique susceptible de détecter différents agents infectieux, notamment en utilisant, simultanément ou successivement, une méthode de détection différente pour chacun, par exemple un anticorps conjugué à la phosphatase alkaline contre p26HIV2 puis un anticorps conjugué à la peroxydase contre HBsAg.

Dans un premier sous-ensemble de modes de mise en oeuvre de la présente invention, où l'on rajoute une (β2GPI)r extérieure au milieu biologique, on peut utiliser, pour la partie β2GPI du complexe, comme forme de β2GPI, la B2'GPI pure ou sous forme de composition protéinique contenant, en particulier, d'autres glycoprotéines ; cette composition peut, en particulier, être celle obtenue par élution d'une colonne d'affinité sur un gel portant des groupes sulfates comme décrit dans la demande de brevet français 2 701 263. Mais on peut aussi utiliser d'autres formes de β2GPI, par exemple obtenue selon le protocole décrit par J. Arvieux et Coil dans Journal of Immunological Methods (1991), 143 p. 223 - 229. La β2GPI carbamylée est susceptible de former certains complexes.

Dans les modes de mise en oeuvre appartenant au premier sous-ensemble précédemment défini, on forme des complexes en rajoutant de la (β2GPI)r à un matériau biologique pour séparer et/ou doser et/ou quantifier des CInV.

Selon un deuxième sous-ensemble de modes de mise en oeuvre du procédé selon l'invention, on utilise la (β2GPI)n naturellement et/ou initialement présente dans un matériau biologique. On sait que le procédé de WO 94/18569 donne des résultats satisfaisants lorsque le matériau biologique renferme des CIV libres, c'est-à-dire non fixés à la β2GPI naturellement présente dans le matériau biologique, lesdits CIV libres étant, par conséquent, susceptibles de se fixer à la β2'GPI rajoutée selon ce procédé. Dans ce procédé, le signal de réponse croît donc en fonction des CIV ayant des sites encore libres ou, éventuellement, libérés par compétition des complexes naturellement présents dans le matériau biologique. En revanche, on peut supposer, dans certains cas, par exemple en début d'infection, que les CIV sont présents en quantité faible par rapport à la (β2GPI)n naturellement présente et que lesdits CIV sont donc majoritairement fixés à la (β2GPI)n sous forme de complexes (β2GPI)n/CI. Le test proposé dans la demande WO 94/18569 peut alors être non significatif, puisque les CIV ainsi complexés peuvent être masqués. Selon ce deuxième sous-ensemble de l'invention, on se propose donc d'observer et/ou de séparer et/ou de détecter et/ou de quantifier des CI se trouvant dans un matériau biologique, notamment dans le cas où ces composés sont en quantité telle, par rapport à la (β2GPI)n habituellement présente, qu'ils soient majoritairement complexés(ables) à au moins une des formes de (β2GPI)n ou encore dans le cas où lesdits CI ne sont pas déplaçables par l'introduction de (β2GPI)r.

Etant donné que, selon ce deuxième sous-ensemble de modes de mise en oeuvre selon l'invention, on n'a ajouté aucune β2GPI, on évite le phénomène de masquage du procédé de WO 94/18569 et on obtient un signal de réponse, qui peut être fonction croissante des quantités de complexe, et donc de CI, contenues dans le matériau biologique, même pour des quantités très faibles de ces CI. Le procédé peut permettre de détecter éventuellement un état initial de la pathologie, alors que le procédé antérieur est plus approprié à l'étude d'un état de pathologie déclarée correspondant, par exemple, à un excès de CI ou à un défaut de (β2GPI)n ou à l'existence naturelle d'une forme de β2GPI non fonctionnelle pour le lien au(x) CI.

Il est à noter que ces deux sous-ensembles de mise en oeuvre ne s'excluent pas l'un l'autre.

Pour mettre en oeuvre le procédé selon l'invention, on peut effectuer la détection des CI sans fixation préalable du complexe β2GPI/CI sur un support ou avec fixation dudit complexe sur. un support par un élément constitutif du complexe ; dans le premier cas, la détection et/ou la quantification s'effectue dans le milieu où le complexe s'est formé, après fixation ou non dudit milieu par une méthode physique, chimique ou biochimique, par exemple sur une surface, ou sans fixation dudit milieu ; dans le deuxième cas, le support peut, avantageusement, être un support solide.

Dans la présente description, on utilise le terme de fixation au support sans préjuger du moment où le complexe est formé.

Selon une variante de l'invention, on retient le complexe β2GPI/CI par l'intermédiaire de la partie β2GPI du complexe, en munissant un support d'un composé se liant à ladite partie β2GPI ; ensuite, la partie du complexe correspondant aux CI est séparée/détectée/quantifiée par tout moyen approprié.

Selon une autre variante de l'invention, on retient le complexe β2GPI/CI par l'intermédiaire de la partie CI dudit complexe en fixant cette dernière à un support muni d'un composé se liant à ladite partie CI du complexe ; ensuite, on détecte et/ou sépare et/ou quantifie la partie β2GPI dudit complexe par tout moyen approprié, avantageusement à l'aide d'anticorps spécifique(s) de la β2GPI, conjugués notamment. La présence native ou le rajout de détergent(s) ou lipide(s) peut aider la fixation de ces anticorps.

Selon l'invention, on peut procéder à la détection par visualisation et/ou numération d'une structure caractéristique du CI notamment à vue, ou en microscopie (notamment optique, électronique, UV) en détectant la (β2GPI)n associée au(x) CI sur ces structures, notamment à l'aidé d'un anticorps spécifique marqué, par exemple conjugué à une molécule enzymatique ou fluorescente. Le(s) CI, complexé(s) ou non, du matériau biologique peuvent être physiquement, chimiquement ou biologiquement fixé(s) à un support, ou en phase liquide (notamment acide, cétonique, alcoolique, paraffinique, ou aldéhydique). On peut aussi utiliser un double marquage spécifique de chaque partie pour détecter le complexe, notamment à l'aide d'anticorps spécifiques de chaque partie, conjugués à 2 traceurs différents, par exemple fluorescents à différentes longueurs d'ondes. Enfin, la détection ou dosage des CI dans le matériau biologique peut se faire à l'aide d'un appareil d'analyse de signaux tels que nombre, volume, taille, forme de particules ou structures, par exemple en cytométrie, de flux notamment.

Selon une autre variante de l'invention, on retient sur un support le complexe β2GPI/CI par l'intermédiaire d'un composé se liant à l'une des parties β2GPI ou CI du complexe, on sépare dudit matériau biologique le complexe fixé sur ledit support et l'on sépare et/ou détecte et/ou on quantifie et/ou on dose ledit complexe (CI/β2GPI) par reconnaissance de l'autre partie du complexe.

Le support est avantageusement un support solide : ce peut être une membrane, par exemple en nitrocellulose, ou une microplaque de titration, par exemple une microplaque ELISA. ou une lame d'observation.

La fixation sur le support du composé se liant à une des parties du complexe, un anticorps par exemple, se fait par réaction de groupes réactifs dudit composé sur les sites réactifs du support. Cette réaction est, de préférence, effectuée à une température comprise entre 0 et 40°C : le composé se liant à une des parties du complexe est, avantageusement, mis dans un tampon ayant un pH compris entre 4,5 et 10,5, de préférence entre 6,5 et 7,5 ; ce tampon peut être avantageusement du type phosphate ou acétate. Le support est avantageusement maintenu en contact avec le tampon contenant le composé à une température comprise entre 0 et 40°C pendant un temps d'incubation compris entre 30 mn et 24 heures. Après incubation, on sépare du support le tampon, qui n'a pas réagi, et on effectue un lavage du support, de préférence avec un tampon identique au précédent, à cette différence près qu'il ne contient pas le composé susmentionné. Il peut être nécessaire de saturer les sites actifs du support, qui n'ont pas réagi avec ledit composé ; dans ce cas, on fait réagir sur ces sites actifs d'autres groupes actifs ; on utilise avantageusement dans ce but, une solution d'albumine sérique, bovine par exemple, ou de caséine et/ou de polyvinyl pyrrolidone et/ou de gélatine et/ou de détergent, simultanément ou successivement. Après réaction, le support est, de préférence, rincé et séché.

Le support, sur lequel est fixé le composé se liant au complexe, est ensuite mis en contact avec un matériau biologique, liquide notamment, contenant le CI à rechercher. On dilue, avantageusement, ce matériau biologique à l'aide d'un tampon donnant un pH compris entre 4,5 et 10,5, de préférence entre 5,6 et 7.5. La réaction sur le support est, de préférence, effectuée à une température comprise entre 0 et 40°C, avantageusement voisine de 37°C, pendant une période dont la durée est comprise entre 30 minutes et 24 heures. On sépare avantageusement ensuite du support la solution contenant le CI, qui n'a pas réagi ; on effectue éventuellement un lavage du support avec une solution saline, de préférence tamponnée.

Dans le cas où la fixation du complexe sur un support s'effectue par la partie β2 GP I du complexe, le composé se liant à la β2 GP I peut être un anticorps reconnaisant la β2 GP I ou une autre protéine, par exemple d'origine virale ou cellulaire, procaryote ou eucaryote, telle une albumine ou un composé biologique, par exemple un acide gras ou un lipide, tel un phospholipide, ou un composé chimique, par exemple le dextran sulfate, l'héparine sulfate ou un détergent. Un radical libre ou activé du support peut lier la β2GPI, quelquefois préférentiellement.

La séparation et/ou le dosage et/ou la quantification du CI du complexe β2GI/CI lié au support par la β2GPI peut se faire par tout moven connu, tel que l'infectiosité, une réaction enzymatique spécifique, un traceur par exemple fluorescent ou radiomarqué, la détection d'acides nucléiques spécifiques par hybridation avec une sonde marquée, une réaction en chaîne obtenue avec une polymérase (technique dite "PCR" ou "polymerase chain reaction"), un dosage, une numération, une visualisation, un procédé optique, une (électro)microscopie. Mais la détection et/ou le dosage se font, de préférence, à l'aide d'un anticorps reconnaissant spécifiquement des protéines des CI à détecter. De façon connue, cet anticorps peut être conjugué à un marqueur enzymatique, par exemple la peroxydase ; l'excès d'anticorps est éliminé par lavage ; on ajoute ensuite, de façon connue, un substrat spécifique de l'enzyme conjugué à l'anticorps, substrat qui se transforme, dans des conditions fixées, en un produit coloré; la formation dudit produit coloré indique la présence du CI recherché et permet un dosage de ce CI. On peut également utiliser un anticorps contre le CI couplé à un marqueur isotopique puis détecté par radio-mesure.

Dans le cas où la fixation au support se fait par l'intermédiaire de la partie CI du complexe, spécifiquement si l'on veut une détection spécifique, on le révèle avec la β2GPI, avantageusement conjuguée à un marqueur. Le CI peut être fixé, soit directement sur le support, soit indirectement, par exemple par l'intermédiaire d'un anticorps. Le marqueur peut, avantageusement, être une enzyme, un produit radioactif. ou un produit fluorescent. La fixation du CI sur le support peut se faire par réaction de groupes réactifs du CI sur les sites réactifs du support lorsque la fixation est directe, ou par fixation d'un composé, par exemple un anticorps, sur les sites réactifs du support et fixation du CI sur ledit composé préalablement fixé sur le support.

La détection de la partie β2 GP I du complexe fixé par l'intermédiaire d'un composé liant la partie CI peut se faire par tout moyen approprié, mais avantageusement à l'aide d'anticorps spécifiques de la β2 GP I, conjugués par exemple.

L'invention a aussi pour objet un support solide pour la mise en oeuvre du procédé ci-dessus défini, caractérisé par le fait qu'il est apte à fixer un des éléments du complexe β2GPI/CI ou une substance apte à fixer un des éléments dudit complexe.

Les exemples ci-après donnés, à titre purement illustratif et non limitatif, permettront de mieux comprendre l'invention. Certains ont été réalisés avec une β2'GPI obtenue selon la demande de brevet français 2 701 263.

### EXEMPLE 1 - Visualisation de Leishmanies

### a) Utilisation de β2'GPI fluorescente

Une suspension de promastigotes de Leishmania infantum obtenue par culture in vitro sur gélose au sang est fixée par l'acétone à 0°C pendant 10 mn sur une lame porte-objet pour observation au microscope optique. La lame est ensuite plongée pendant 5 mn dans un tampon phosphate, ci-après désigné par "TP", contenant 0,01 M de phosphates monosodique et disodique et 0,15 M de chlorure de sodium à un pH de 7,2 ± 0.1. La β2'GPI est couplée à la fluorescéine ; elle est ensuite déposée à une concentration de 20 µg/ml et 2 µ g / m l sur la suspension fixée de promastigotes, puis laissée pendant 30 mn à 20°C en chambre humide. L'élimination de la β2'GPI fluorescente non fixée se fait en plongeant les lames pendant 5 mn dans deux bains successifs de TP. La fluorescence des parasites, notamment de leur pourtour, témoigne de la formation de complexes et permet de les visualiser et identifier.

### b) Utilisation d'un autre marquage de la β2'GPI.

Dans une autre expérience, la β2'GPI, couplée à la phosphatase alcaline désignée par "DAP" ci-après, selon Avrameas (Immunochem. (1969) 8, p. 43 - 52), a réagi avec les parasites fixés sur lame par l'acétone. Le tampon phosphate a été remplacé de façon à ne pas inhiber la phosphatase alcaline.

Le protocole réalisé à été le suivant, à température du laboratoire : réhydratation des parasites pendant 5 mn en sérum physiologique ; incubation de la β2'GPI sous forme DAP en dilution dans du tampon acétate contenant 0,05 % de détergent commercialisé sous le nom de "Triton X100®", désigné ci-après "TX100", à 37°C pendant 30 minutes ; 1 lavage en 50 mM Tris-HCl (pH = 8.2), 50 mM NaCl pendant 10 mn, 1 lavage en sérum physiologique pendant 10 mn, révélation de l'activité phosphatase alcaline par le système vendu sous la dénomination "fast read TR/ naphtol". Dans ces conditions, l'observation au microscope optique montre que la β2'GPI réagit encore significativement à une concentration de 1 µg/ml.

### c) Essais en fluorescence sur plusieurs souches de Leishmanies.

Différentes speciès de Leishmanies ont été utilisées pour mettre en évidence l'interaction entre la β2'GPI fluorescente et les Leishmanies. On a ainsi teste : L. Infantum, L. Marjon, L. Guyanensis, L. Tronicer, L. Donorium, L. Braziliensis. Toutes ont montré une réaction significative avec la β2'GPI fluorescente avec addition de 0,05% de "TX100" et de 0,25M de NaCl pendant la réaction.

### EXEMPLE 2

La même manipulation qu'à l'exemple 1 a été effectuée avec Toxoplasma gondji et la fluorescence du parasite a été observée avec au moins 20 µg/ml de β2'GPI fluorescente.

Pour ces exemples 1 et 2, d'autres expériences ont permis d'augmenter la sensibilité de détection des Leishmania et Toxoplasmes par la β2'GPI fluorescente, par l'addition de 0,05% de "TX100" et de 0,25 M NaCl dans le tampon d'incubation.

Des mélanges de parasites (Leishmanies) et de GR de lapin ou humain ont été fixés sur lame par l'acétone puis mis en réaction dans les conditions améliorées ci-dessus. L'image observée montre un net contraste entre les parasites fortement fluorescents et les érythocytes très faiblement marqués.

A titre de contrôle, un autre réactif fluorescent que la β2'GPI, composé de F(ab')c de lapins immunopurifiés et couplés à la fluorescéine, spécifiques des IgG de chèvres et n'ayant donc à priori aucune spécificité pour les parasites, a été réalisé dans ces conditions améliorées ci-dessus, à des concentrations identiques, en protéines et Fluorescéine à celles de la β2'GPI fluorescente. Ce réactif, ainsi d'ailleurs qu'une solution de Fluorescéine pure de concentration comparable, utilisé dans les mêmes conditions, ne donne qu'une fluorescence très faible sur des parasites, de l'ordre du "bruit de fond".

### EXEMPLE 3

La même manipulation qu'à l'exemple 1 a été effectuée sur Entamoeba histolytica et la fluorescence du parasite a été observée avec au moins 20 µg/ml de β2'GPI fluorescente.

### EXEMPLE 4 Détection d'antigènes solubles de Leishmania infantum

Le support utilisé est une plaque de microtitration, de type micro-ELISA, à 96 puits et à fond plat, sur lequel sont fixés des antigènes solubles de Leishmania infantum. Ce support sensibilisé est distribué par la société BIOKEMA-Affinity Products (Suisse).

On prépare une solution de β2'GPI marquée à la peroxydase ayant une concentration de 10 µg/ml dans un tampon acétate contenant 0,05 mole/l d'acide acétique et d'acétate de sodium, 0,01 % d'albumine bovine sérique et 0,05 % en poids de "TX100", ce tampon ayant un pH de 5,6 ± 0,1. On ajoute 100 µl par puits de la solution précédente et on laisse incuber pendant 1 h 30 à 37°C. A la suite de cette incubation, le contenu des puits de la plaqué est aspiré. On introduit 300 µl de tampon phosphate dans chaque puits et après un temps de contact de 2 minutes, on aspire le tampon : cette opération de lavage est renouvelée 3 fois.

On ajoute par puits 100 µl d'une solution d'o-phénylène-diamine OPD, 2 HCl dans un tampon citrate de sodium. On laisse incuber pendant 30 minutes à température ambiante, puis on arrête la réaction en rajoutant à chaque puits 50 µl de H₂SO₄, 2N. On mesure en unités de densité optique (UDO) l'absorbance à 492 nm obtenue en fin de réaction, cette mesure étant faite à l'aide d'un robot lecteur de plaque.

Les résultats obtenus démontrent une reconnaissance des antigènes solubles de Leishmania infantum par la β2'GPI. Les mêmes résultats sont obtenus en utilisant de la β2'GPI non marquée, la liaison étant ensuite révélée à l'aide d'un anticorps monoclonal anti-β2'GPI marqué à la peroxydase.

### EXEMPLE 5 Liaison de la β2'GPI sur des antigènes de Borrelia

On a utilisé une membrane de nitrocellulose sur laquelle sont transférés, après électrophorèse, des antigènes de Borrelia (don de I. Nilsson).

On fait réagir pendant 1 h 30 à température ambiante sur ladite membrane environ 40 ng de β2'GPI, couplée à de la phosphatase alcaline, en solution dans 1 ml de tampon - acétate (acide acétique/acétate de sodium) à 0.05 mole/l, contenant 0,1 % en poids de gélatine et 0,5 % de "TX100", ce tampon ayant un pH de 5.6 ± 0,1. On rince ensuite une fois avec un tampon acétate à 0.05. mole/l, contenant 0,05 % en poids de "TX100" et ayant un pH de 5,6 ± 0,1. On rince ensuite deux fois avec du tampon phosphate contenant 0,01 mole/l de phosphates monosodique et disodique, 0,15 mole/l de chlorure de sodium, 0,05 % en poids de "TX100", ce tampon ayant un pH de 7,00 ± 0,1. On révèle l'activité de la phosphatase alcaline en présence de nitro-blue-tétrazolium (NBT) et de 5-bromo-4-chloro-3-indolyl phosphate (BCIP), dans une solution à 50 mM de tri(hydroxyméthyl) aminométhane, neutralisé par l'acide chlorydrique (TrisHCl) jusqu'à pH 8,8 et à 0,1 M de NaCl. On voit sur la membrane que la β2'GPI permet de révéler la présence d'antigènes de Borrelia Afzelii, notamment ceux qui pourraient indiquer une pathogénicité, par exemple p39, osp B, osp A, osp C, selon les désignations indiquées dans la publication de Ingrid Nilsson et autres (Serodiagn. Immunother. Infect. Disease, 1993, 5, p. 245-250). Dans les mêmes conditions, il n'y a pas de réaction avec la phosphatase alcaline seule.

### EXEMPLE 6 - Détection de l'antigène HBs du virus de l'hépatite B

Le support utilisé est une plaque de microtitration de type micro-ELISA à 96 puits et à fond plat, commercialisée par la société "DYNATECH". On prépare une solution de protéines recombinantes p26-HIV2 ROD fournies par la société "TRANSGENE" ayant une concentration de 2 µg/ml, dans un tampon phosphate contenant 0,01 mole/l de phosphates monosodique et disodique et 0.15 mole/l de chlorure de sodium et ayant un pH de 7,00 ± 0,05. On dépose 100 µl de cette solution au fond de chaque puits de la microplaque. Celle-ci est ensuite incubée à + 4°C pendant 18 heures. Le liquide de chaque puits est ensuite aspiré. On introduit ensuite dans chaque puits 300 à 400 µl de tampon phosphate décrit ci-dessus contenant le détergent "TX100" à 0.05 % en poids. On laisse en contact avec le support pendant 3 minutes et on aspire le tampon ; cette opération de lavage est effectuée trois fois.

On a utilisé trois échantillons de sérum de donneurs sains et quatre échantillons de sérum de malades infectés par le virus de l'hépatite B. L'échantillon sérique est dilué dix, cent ou mille fois dans du tampon acétate contenant 0.05 mole/l d'acide acétique et d'acétate de sodium. 0,5 % en poids de "TX100", 0.01 % en poids d'albumine sérique bovine, ayant un pH de 5,6 ± 0,1. On dépose 100 µl de solution au fond de chaque puits de la plaque préparée comme ci-dessus. La plaque est incubée à 37°C pendant une période de 90 minutes. Après cette incubation, on effectue un lavage en introduisant dans chaque puits 300 µl de tampon phosphate contenant du "TX100" à 0.05 % en poids ; on laisse en contact pendant 2 minutes et on aspire la solution de tampon ; on renouvelle quatre fois cette opération de lavage.

On ajoute ensuite, par puits, 100 µl d'une solution d'anticorps monoclonal spécifique contre l'antigène HBs du virus de l'hépatite B conjugué à la peroxydase. On laisse la plaque incuber à 37°C pendant 60 minutes. A la suite de cette incubation, le contenu des puits de la plaque est aspiré. On introduit dans chaque puits 300 µl de tampon phosphate, contenant 0,05 % en poids de "'TX100" et, après un temps de contact de 2 minutes, on aspire le tampon ; cette opération de lavage est renouvelée cinq fois.

On ajoute, par puits, 100 µl d'une solution de d'o-phénylène diamine, 2HCL dans un tampon de citrate de sodium. On laisse incuber pendant 30 minutes à température ambiante, puis on arrête la réaction en rajoutant à chaque puits 50 µl de H₂SO₄, 2N. On mesure l'absorbance à 492 nm obtenue en fin de réaction à l'aide d'un robot lecteur de plaque.

La moyenne des absorbances obtenues pour chaque malade ou donneur est donnée dans le tableau 1 (en unités de densité optique multipliées par 1 000). Pour les quatre sujets malades et non pour les trois sujets sains, l'AgHBs a été efficacement révélé.

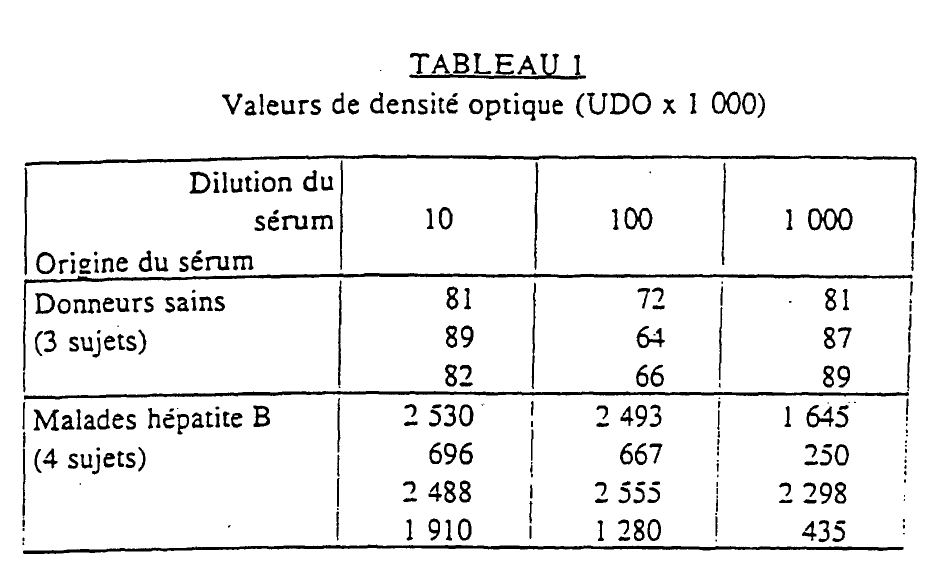

### EXEMPLE 7

On a utilisé la même procédure que pour l'exemple 6, à cette différence près que, sur le support, est fixé un anticorps monoclonal dirigé contre la β2GPI, appelé "8C3" (don de J. Arvieux). Des résultats similaires ont été obtenus.

Pour ces deux exemples 6 et 7, d'une part, on a montré que de la β2GPI s'était fixée au support en la révélant par des anticorps spécifiques, d'autre part, on a pu inhiber la fixation d'un complexe (β2 GP I/AgHBs) présent dans un sérum de malade atteint d'hépatite B en bloquant sa fixation à un support préparé pour fixer la β2 GP I grâce à une pré-incubation du sérum de malade à 37°C pendant 30 minutes avec des anticorps dirigés contre la β2 GP I. Ces résultats indiquent que l'AgHBs du virus de l'hépatite B peut être reconnu par l'intermédiaire de la β2 GP I présente dans le plasma, celle-ci étant, dans ces essais, fixée par la p26-HIV2 ROD ou un anticorps spécifique.

### EXEMPLE 8

Avec la même procédure que celle utilisée pour l'exemple 6, les résultats ont été positifs pour la détection d'antigènes de surface portés par le parasite LEISHMANIE, extrait d'un tissu infecté.

### EXEMPLE 9 (exemple ne faisant pas partie de la présente invention) - Formation d'un complexe β2GPI/cellule de lignées lymphocytaires immortalisées

La β2GPI fluorescente est incubée avec des cellules de la lignée "CEM"(10⁶/ml) prérincées 2 fois en tampon PBS pendant 1 h à 37°C. Après 3 lavages en en tampon PBS, les cellules sont fixées par 2 % de paraformaldéhyde puis analysées en cytofluoromètrie de flux. L'analyse des spectres de fixation montre la présence de la β2GPI sur la majorité des cellules de manière dose dépendante, de 10 à 100 µg /ml initial.

Cette fixation a été observée pour d'autres lignées immortalisées telles des lignées T : SVPT1, MOLT4, ou monocytaires : THPI, U937, au contraire des lymphocytes normaux du sang périphérique de donneurs sains, isolés par centrifugation sur un coussin d'une solution, commerciale "Ficoll®, puis rincés en PBS.

### EXEMPLE 10 - Fixation d'un complexe (β2GPI)n/HBsAg.

Dans cet exemple, le composé liant la partie (β2GPI)n est de l'albumine sérique humaine (HSA) purifiée selon le procédé décrit dans WO93/21228. Des plaques ELISA (Nunc Maxisorb) sont préchargées avec une solution de 1 % de HSA en 0,1 M Trisglycin pH 8,8, puis lavées extensivement en PBS pH 7,2.

Un sérum HBsAg+ ou un sérum de donneur sain sont incubés à la dilution du millième en acétate de sodium (50 mM, pH 5,6) pendant 1 heure à 37°C et l'expérience conduite comme dans l'exemple 6 mais en l'absence de "TX100".

Le sérum de donneur ou les contrôles sans sérum ne donnent aucun signal notable (< 0,05 udo). Le sérum HBsAg donne un signal significatif (= 1,5 udo). HBsAg est donc capturé. Si la dilution sérique est incubée :
a) en présence de 800 µg/ml de HSA, le signal est abaissé de plus de 80 % indiquant que le lien se fait bien à la HSA fixée au support ;
b) en présence de 4 µg/ml de β2GPI, soit seulement 20 fois la quantité de β2 interne provenant du sérum, le signal est abaissé de près de 40 %, signifiant qu'une partie des complexes internes se fixe au support.

Si une expérience similaire est faite sans préchargement des plaques, on observe que l'HBs est révélé aussi, inhibé à 97 % par une préincubation en présence de HSA et à 65 % en présence de β2GPI rajoutée.

### EXEMPLE 11 - Formation d'un complexe de (β2'GPI)r avec des protéines de Mycoplasma penetrans

On a procédé comme dans l'exemple 5 sur des bandes de nitrocellulose contenant quelques microgrammes de CI traité par 2 % d'octyl glucoside, saturées selon le procédé EURIS - 14, rue du Chapeau Rouge - 34500 BEZIERS (France). La β2GPI marquée sous forme de DAP est nettement fixée à certains antigènes détectés par un sérum immunospécifique ou par un sérum spécifique de la p 35, notamment donc fortement les antigènes à 35, 40 et 45 Kd et légèrement l'antigène 65 Kd.

### EXEMPLE 12 - Fixation à l'anatoxine tétanique de (β'2GPI)r

De l'anatoxine tétanique (ELOCORIDE, Behring) a été transférée après électrophorèse en gel d'acrylamide à 10 % sur nitrocellulose, à raison d'environ 0,5 à 2 µg/mm².

Après saturation des membranes selon EURIS, puis 4 rinçages avec une solution à 50 mM de Tris HCl (pH = 8,2), 50 mM NaCl, et 1 rinçage en 50 mM Hepes - NaOH (pH 6,8 ± 0,1), 100 ng de DAP dans 1 ml de ce dernier tampon ont été ajoutés sur les membranes et incubés pendant une heure à 25°C en présence ou non de 10 µg de cardiolipine, désignée par "CL". Les membranes ont été rincées 4 fois par 1 ml de 50 mM Tris-HCl (pH = 7,2), 150 mM NaCl.

Après révélation comme dans l'exemple 5, la zone contenant l'antigène tétanique est la seule colorée en présence de CL. En l'absence de CL, la réaction n'a pas lieu, ce qui indique la nécessité de la présence de CL pour former le complexe dans ces conditions.

Le rôle des détergents et/ou lipides dans la formation du complexe β2GPI/CI a été plus amplement illustré par les expériences ci-après rapportées.

100 à 200 ng de protéines virales, en solution dans de l'eau contenant 7.5 g de Tris et 14,4 g de glycine par litre et 20 % de méthanol, sont filtrés lentement à travers 2 à 4 mm2 d'une membrane de nitrocellulose (BA84 commercialisée par "Schleicher et Schull"). Les membranes sont saturées par le même tampon sans méthanol contenant 1% de HSA obtenu comme indiqué dans l'exemple 10. Le support est ensuite rincé extensivement avec quatre brefs prélavages en 50 mM Tris-HCl (pH = 8,2), 50 mM NaCI puis incubé de 1 à 4 heures avec de la β2GPI dans un milieu de réaction, enfin lavé par des postrinçages; la β2GPI est révélée, comme indiqué ci-après.

De façon générale, on constate, qu'en l'absence de lipide et/ou de détergent, la B2GPI est fixée à l'albumine utilisée pour saturation et non sur certaines des protéines virales, cependant qu'en présence de détergent, l'inverse est observé, à savoir que la β2GPI n'est plus fixée sur l'albumine mais sur certaines protéines virales. Un phospholipide tel que la cardiolipine provoque une telle fixation.

Plus précisément, on a constaté les résultats suivants :
A) - On effectue les étapes suivantes : réaction avec 50 ng par ml de DAP; 4 postrinçages en 50 mM Tris HCI (pH = 8,2), NaCI 50 mM; révélation de la DAP en 0,1 M Tris HCI (pH = 8,8), NBT et BCIP selon les conditions recommandées par Merck.
   En l'absence de détergent dans le milieu de réaction (acétate de Na, 50 mM, pH = 5,6), les recombinants p18 et p25 HIV₁, p26 HIV₂ (TRANSGENE) ne réagissent pas (zone blanche du support), alors que le recombinant gp160 de HIV fixe légèrement DAP et que l'albumine humaine réagit aussi (fond coloré). Par contre, en présence de 0,5 % de "TX100" ou de "Triton 405®" ou de 0,2 % de "Tween 20®", l'albumine ne montre plus de réaction, cependant que p26 HIV₂, p18 HIV₁ montrent une forte réaction, gp160 HIV₁ une réaction supérieure à celle sans détergent et p25 HIV₁ une faible réaction.
B) Dans les mêmes conditions, mais en milieu de réaction différent 50 mM Tris HCI (pH = 8,2), seule la p18 HIV₁ réagit faiblement en présence de détergent.
C) - On effectue les étapes suivantes : 1 prélavage en Hepes 50mM, NaOH, (pH = 6,8); réaction pendant 1 Heure dans ce même tampon avec 100 ng/ml de DAP, en présence ou non de 10 µg/ml de cardiolipine ;
   4 postrinçages en 0,15 M NaCl 50 mM Tris HCI (pH = 7,2), 150 mM NaCl et un postrinçage en 0,15 M NaCl et révélation comme en A.
   Aucune des 4 protéines de HIV ne réagit sans cardiolipine et les 4 réagissent en sa présence.
D) On effectue les étapes suivantes : 1 heure de réaction 50 mM Tris HCI (pH = 7,6) en présence de 1 µg/ml de β2'GPI, et 20 µg/ml de CL, 4 lavages dans le tampon de réaction, révélation de la β2'GPI par 1 heure d'incubation en présence de 54 µg/ml d'anticorps monoclonal 8C3, couplé à la phosphatase alcaline, en solution dans le même tampon additionné de 0,15 M de NaCl et 0,05 % de "Tween 20" ; puis 4 rinçages par la même solution et 1 rinçage par 0,15 M NaCl et révélation comme en A.

Les 4 protéines virales réagissent et la p25 HIV₁ réagit fortement, alors que les contrôles sans β2'GPI ou sans CL ne montrent aucune réaction visible.

Ces exemples, entre autres, indiquent qu'un détergent et/ou un lipide, notamment un phospholipide, peuvent favoriser l'interaction avec les protéines virales, si ce n'est participer à ladite interaction, et que le lien à l'albumine est d'une autre nature puisque sensible aux détergents utilisés.

On a noté aussi que détergent(s) ou phospholipide(s) peuvent être ajoutés dans un stade préalable à la présence de β2GPI. On notera que le taux de phospholipides sur les protéines virales de HTV₁ est connu pour être élevé et qu'on a décrit un lien entre β2GPI et HBsAg recombinant en présence de détergent. Il en résulte que, pour certains CI et dans certaines conditions de milieu, un détergent ou un lipide est nécessaire pour la formation du complexe β2GPI/CI.

## Revendications

1. Procédé de séparation et/ou de détection et/ou de quantification dans un matériau biologique de composés infectieux (CI), en particulier protéiniques, choisis dans le groupe formé par
- des composés constitutifs spécifiques et identifiables d'un agent infectieux,
- des structures qui comprennent
- des agents infectieux endogènes ou exogènes, complets ou incomplets,
- les métabolites spécifiques et identifiables de ces agents infectieux,
- des assemblages spécifiques et identifiables de ces agents infectieux présentant des propriétés spécifiques des agents infectieux,
- des composés identifiables et spécifiques d'un agent infectieux induits dans l'organisme par les agents infectieux définis ci-dessus,
**caractérisé par le fait que** l'on sépare et/ou détecte et/ou quantifie un complexe β2GPI/CI, dans lequel β2GPI est la β2 glycoprotéine I et CI un composé infectieux, identifiable par tout moyen adapté, choisi dans le groupe formé par :
a) (β2GPI)n/CI, (β2GPI)n étant la β2 glycoprotéine I naturellement présente dans le matériau biologique, CI étant un composé viral CIV ou un composé non viral CInV,
b) (β2GPI)r/CInV, (β2GPI)r étant de la β2 glycoprotéine I rajoutée au matériau biologique et CInV étant un composé infectieux non viral.

2. Procédé selon la revendication 1 **caractérisé par le fait que** l'on sépare et/ou détecte et/ou quantifie les CI par la reconnaissance, à l'aide d'une substance se liant à la β2GPI, du complexe β2GPI/CI.

3. Procédé selon l'une des revendications 1 ou 2, **caractérisé par le fait que** l'on sépare et/ou détecte et/ou quantifie le complexe β2GPI/CI dans le milieu où il s'est formé, sans fixation préalable du complexe sur un support, avec ou sans fixation dudit milieu.

4. Procédé selon l'une des revendications 1 ou 2, **caractérisé par le fait que** l'on retient l'un des éléments constitutifs du complexe β2GPI/CI sur un support.

5. Procédé selon la revendication 4, **caractérisé par le fait que** le support est un support solide.

6. Procédé selon la revendication 5, **caractérisé par le fait que** le support solide utilisé est une membrane, une microplaque de titration ou une lame d'observation.

7. Procédé selon l'une des revendications 4 à 6, **caractérisé par le fait que** l'on retient le complexe β2GPI/CI sur le support par l'intermédiaire de l'élément β2GPI du complexe.

8. Procédé selon l'une des revendications 4 à 6, **caractérisé par le fait que** l'on retient le complexe β2GPI/CI sur le support par l'élément CI du complexe.

9. Procédé selon la revendication 8, **caractérisé par le fait que** l'on réalise la fixation de l'élément CI sur le support avant formation du complexe β2GPI/CI, que l'on forme ensuite ledit complexe, puis que l'on sépare et/ou détecte et/ou quantifie l'élément β2GPI du complexe.

10. Procédé selon l'une des revendications 8 ou 9, **caractérisé par le fait que** l'on sépare et/ou détecte et/ou quantifie, à l'aide d'une substance se liant à la β2GPI du complexe, le(s) CI du matériau biologique, physiquement, chimiquement ou biochimiquement fixé(s) à un support.

11. Procédé selon la revendication 1 ou 4, **caractérisé par le fait que** l'on retient sur un support le complexe β2GPI/CI par l'intermédiaire d'un composé se liant à l'une des parties β2GPI ou CI du complexe, que l'on sépare dudit matériau biologique le complexe fixé sur ledit support et que l'on sépare et/ou détecte et/ou quantifie ledit complexe par reconnaissance de l'autre partie du complexe.

12. Procédé selon la revendication 11, **caractérisé par le fait que** l'on retient le complexe sur le support par l'intermédiaire d'un composé se liant à la partie β2GPI du complexe.

13. Procédé selon la revendication 11, **caractérisé par le fait que** l'on retient le complexe sur le support par l'intermédiaire d'un composé se liant à la partie CI du complexe.

14. Procédé selon la revendication 2, **caractérisé par le fait que** le composé se liant à la β2GPI est choisi dans le groupe formé par un anticorps dirigé contre la β2GPI, une autre protéine, un composé biologique ou chimique se fixant à la β2GPI.

15. Procédé selon la revendication 14, **caractérisé par le fait que** la protéine reconnaissant la β2GPI est la protéine p26-HIV₂ ROD.

16. Procédé selon la revendication 1, **caractérisé par le fait que** le CI du complexe fait partie du groupe formé par HIV1, HIV2, HSV, HBV et des particules ou protéines d'origine virale.

17. Procédé selon la revendication 1, **caractérisé par le fait que** le CI du complexe fait partie du groupe formé par les bactéries, les parasites, les mycoplasmes et les champignons.

18. Procédé selon la revendication 7, **caractérisé par le fait que** l'on sépare et/ou détecte et/ou quantifie le CI du complexe β2GPI/CI, fixé sur le support, par infectiosité, par une réaction enzymatique spécifique, par un traceur fluorescent ou radiomarqué, par la détection d'acides nucléiques par hybridation avec une sonde marquée ou par une réaction de polymérase en chaîne (PCR) ou encore à l'aide d'anticorps spécifique(s) du CI.

19. Procédé sélon Tune des revendications 1 à 18, **caractérisé par le fait que** l'on utilise, comme forme de β2GPI, la β2'GPI sous forme pure ou non.

20. Procédé selon l'une des revendications 1 à 19, **caractérisé par le fait que** la β2GPI utilisée pour constituer le complexe avec le(s) CI est marquée, la détection et/ou quantification du complexe s'effectuant grâce audit marquage.

21. Procédé selon la revendication 20, **caractérisé par le fait que** le marquage de la β2GPI est réalisé au moyen d'une enzyme, d'un produit radioactif ou d'un produit fluorescent.

22. Procédé selon la revendication 6, **caractérisé par le fait que** l'on réalise la fixation de l'élément β2GPI sur le support avant formation du complexe β2GPI/CI, que l'on forme ensuite ledit complexe, puis que l'on détecte et/ou quantifie l'élément CI du complexe.

23. Procédé selon l'une des revendication 1 à 22, **caractérisé par le fait que** l'un au moins des éléments du complexe est d'origine animale ou produit par génie génétique et/ou chimique.

## Patentansprüche

1. Verfahren zur Abtrennung und/oder Detektion und/oder quantitativen Bestimmung von infektiösen Verbindungen (Cl), insbesondere von Proteinverbindungen, in einem biologischen Material, ausgewählt unter
- spezifischen und identifizierbaren Verbindungsbestandteilen eines infektiösen Agens,
- den Strukturen, welche
- endogene oder exogene, vollständige oder unvollständige infektiöse Agenzien,
- spezifische und identifizierbare Metaboliten dieser infektiösen Agenzien,
- spezifische und identifizierbare Zusammensetzungen dieser infektiösen Agenzien, welche die spezifischen Eigenschaften der infektiösen Agenzien aufweisen,
umfassen
- identifizierbaren und spezifischen Verbindungen eines infektiösen Agens, die mittels eines wie unten definierten infektiösen Agens in den Organismus eingeführt werden,
**dadurch gekennzeichnet, dass** man einen Komplex β2GPI/CI, worin β2GPI für das β2-Glycoprotein I steht und Cl für eine infektiöse Verbindung steht, der durch jedes geeignete Verfahren identifiziert werden kann und ausgewählt ist unter
a) (β2GPI)n/CI, worin (β2GPI)n für das β2-Glycoprotein I, das natürlicherweise im biologischen Material vorliegt, steht, wobei es sich bei Cl um eine virale Verbindung CIV oder um eine nichtvirale Verbindung CInV handelt,
b) (β2GPI)r/CInV, worin (β2GPI)r für das dem biologischen Material hinzugefügte β2-Glycoprotein I steht und CInV für eine nichtvirale infektiöse Verbindung steht,
abtrennt und/oder detektiert und/oder quantitativ bestimmt.

2. Verfahren nach Anspruch 1, **dadurch gekennzeichnet, dass** man die Cl durch Erkennung des Komplexes β2GPI/CI mit Hilfe einer Substanz, die an das β2GPI bindet, abtrennt und/oder detektiert und/oder quantitativ bestimmt.

3. Verfahren nach einem der Ansprüche 1 oder 2, **dadurch gekennzeichnet, dass** man den Komplex β2GPI/CI in dem Milieu, in dem er sich bildet, ohne vorausgegangene Fixierung des Komplexes auf einem Träger, mit oder ohne Fixierung des Milieus abtrennt und/oder detektiert und/oder quantitativ bestimmt.

4. Verfahren nach einem der Ansprüche 1 oder 2, **dadurch gekennzeichnet, dass** man einen der Bestandteile des β2GPI/CI-Komplexes auf einem Träger festhält.

5. Verfahren nach Anspruch 4, **dadurch gekennzeichnet, dass** es sich bei dem Träger um einen festen Träger handelt.

6. Verfahren nach Anspruch 5, **dadurch gekennzeichnet, dass** es sich bei dem verwendeten festen Träger um eine Membran, eine Mikrotiterplatte oder ein Beobachtungsplättchen handelt.

7. Verfahren nach einem der Ansprüche 4 bis 6, **dadurch gekennzeichnet, dass** man den β2GPI/CI-Komplex mittels des Komplexbestandteils β2GPI auf dem Träger festhält.

8. Verfahren nach einem der Ansprüche 4 bis 6, **dadurch gekennzeichnet, dass** man den Komplex β2GPI/CI mittels des Komplexbestandteils Cl auf dem Träger festhält.

9. Verfahren nach Anspruch 8, **dadurch gekennzeichnet, dass** man den Bestandteil Cl auf dem Träger vor der Bildung des Komplexes β2GPI/CI fixiert, anschließend den Komplex herstellt und danach den Komplexbestandteil β2GPI abtrennt und/oder detektiert und/oder quantitativ bestimmt.

10. Verfahren nach einem der Ansprüche 8 oder 9, **dadurch gekennzeichnet, dass** man mittels einer Substanz, die an das β2GPI des Komplexes bindet, den oder die Cl des biologischen Materials, welche(r) physikalisch, chemisch oder biochemisch an einen Träger gebunden ist/sind, abtrennt und/oder detektiert und/oder quantitativ bestimmt.

11. Verfahren nach einem der Ansprüche 1 oder 4, **dadurch gekennzeichnet, dass** man den Komplex β2GPI/CI mittels einer Substanz, die an einen der Bestandteile β2GPI oder Cl des Komplexes bindet, auf einem Träger festhält, aus dem biologischen Material den auf dem Träger fixierten Komplex abtrennt und den Komplex durch Erkennung des anderen Komplexbestandteils abtrennt und/oder detektiert und/oder quantitativ bestimmt.

12. Verfahren nach Anspruch 11, **dadurch gekennzeichnet, dass** man den Komplex mittels einer Verbindung, die an die β2GPI-Gruppe des Komplexes bindet, auf dem Träger festhält.

13. Verfahren nach Anspruch 11, **dadurch gekennzeichnet, dass** man den Komplex mittels einer Verbindung, die an die CI-Gruppe des Komplexes bindet, auf dem Träger festhält.

14. Verfahren nach Anspruch 2, **dadurch gekennzeichnet, dass** die Verbindung, die an das β2GPI bindet, ausgewählt ist unter einem Antikörper gegen das β2GPI, einem anderen Protein und einer biologischen oder chemischen Verbindung, die an das β2GPI bindet.

15. Verfahren nach Anspruch 14, **dadurch gekennzeichnet, dass** es sich bei dem das β2GPI erkennenden Protein um das Protein p26-HIV₂ ROD handelt.

16. Verfahren nach Anspruch 1, **dadurch gekennzeichnet, dass** das Cl des Komplexes ausgewählt ist unter HIV1, HIV2, HSV, HBV und Teilchen oder Proteinen viralen Ursprungs.

17. Verfahren nach Anspruch 1, **dadurch gekennzeichnet, dass** das Cl des Komplexes ausgewählt ist unter Bakterien, Parasiten, Mycoplasmen und Pilzen.

18. Verfahren nach Anspruch 7, **dadurch gekennzeichnet, dass** man das Cl des β2GPI/CI-Komplexes, der auf einem Träger gebunden ist, durch Infektiösität, durch eine spezielle enzymatische Reaktion, mittels eines Fluoreszenz- oder eines radiomarkierten Indikators, durch Nukleinsäuren-Detektion, durch Hybridbildung mittels einer markierten Sonde oder durch Polymerasenkettenreaktion (PCR) oder auch mittels spezifischer Cl-Antikörper abtrennt und/oder detektiert und/oder quantitativ bestimmt.

19. Verfahren nach einem der Ansprüche 1 bis 18, **dadurch gekennzeichnet, dass** man β2GPI in Form von reinem oder nichtreinem β2'GPI verwendet.

20. Verfahren nach einem der Ansprüche 1 bis 19, **dadurch gekennzeichnet, dass** das zur Bildung des Komplexes mit dem/den CI verwendete β2GPI markiert ist und die Detektion und/oder quantitative Bestimmung des Komplexes auf der Markierung beruht.

21. Verfahren nach Anspruch 20, **dadurch gekennzeichnet, dass** die Markierung des β2GPI mittels eines Enzyms, eines radioaktiven Produktes oder eines fluoreszierenden Produkt erfolgt.

22. Verfahren nach Anspruch 6, **dadurch gekennzeichnet, dass** man den Bestandteil β2GPI vor der Bildung des Komplexes β2GPI/CI auf dem Träger fixiert, anschließend den Komplex herstellt und dann den Komplexbestandteil Cl detektiert und/oder quantitativ bestimmt.

23. Verfahren nach einem der Ansprüche 1 bis 22, **dadurch gekennzeichnet, dass** wenigstens einer der Komplexbestandteile tierischen Ursprungs ist oder genetisch und/oder chemisch hergestellt wird.

## Claims

1. Process for separating and/or detecting and/or quantifying, in a biological material, infectious compounds (IC's), in particular proteinaceous infectious compounds, which are selected from the group formed by
- specific and identifiable constitutive compounds of an infectious agent,
- structures which comprise
- endogenous or exogenous, complete or incomplete infectious agents,
- the specific and identifiable metabolites, of these infectious agents,
- specific and identifiable assemblies of these infectious agents which exhibit specific properties of the infectious agents,
- specific and identifiable and compounds of an infectious agent which are infectious agents induced in the organism by the abovedefined,
**characterized in that** a β2GPI/IC complex, in which β2GPI is β2 glycoprotein I and IC is an infectious compound, which is identifiable by any appropriate means, and which is selected from the group formed by:
a) (β2GPI)n/IC, with (β2GPI)n being the β2 glycoprotein I which is naturally present in the biological material and IC being a viral compound, VIC, or a non-viral compound, nVIC,
b) (β2GPI)a/nVIC, with (β2GPI)a being the β2 glycoprotein I which is added to the biological material and nVIC being a non-viral infectious compound,
is separated and/or detected and/or quantified.

2. Process according to Claim 1, **characterized in that** the IC's are separated and/or detected and/or quantified by the recognition, using a substance which binds to β2GPI, of the β2GPI/IC complex.

3. Process according to either Claim 1 or 2, **characterized in that** the β2GPI/IC complex is separated and/or detected and/or quantified in the medium in which it is formed, without prior attachment of the complex to a support, with or without attachment of the said medium.

4. Process according to either Claim 1 or 2, **characterized in that** one of the elements contained in the β2GPI/IC complex is held on a support.

5. Process according to Claim 4, **characterized in that** the support is a solid support.

6. Process according to Claim 5, **characterized in that** the solid support which is employed is a membrane, a microtitration plate or a microscope slide.

7. Process according to one of Claims 4 to 6, **characterized in that** the β2GPI/IC complex is held on the support by way of the β2GPI element of the complex.

8. Process according to one of Claims 4 to 6, **characterized in that** the β2GPI/IC complex is held on the support by means of the IC element of the complex.

9. Process according to Claim 8, **characterized in that** the IC element is attached to the support before the β2GPI/IC complex is formed, **in that** the said complex is then formed, and **in that** the β2GPI element of the complex is then separated and/or detected and/or quantified.

10. Process according to either Claim 8 or 9, **characterized in that** the IC('s) of the biological material, which is/are physically, chemically or biochemically attached to a support, are separated and/or detected and/or quantified using a substance which binds to the β2GPI of the complex.

11. Process according to Claim 1 or 4, **characterized in that** the β2GPI/IC complex is held on a support by means of a compound which binds to either the β2GPI part or the IC part of the complex, **in that** the complex, which is attached to the said support, is separated from the said biological material, and **in that** the said complex is separated and/or detected and/or quantified by recognition of the other part of the complex.

12. Process according to Claim 11, **characterized in that** the complex is held on the support by way of a compound which binds to the β2GPI part of the complex.

13. Process according to Claim 11, **characterized in that** the complex is held on the support by way of a compound which binds to the IC part of the complex.

14. Process according to Claim 2, **characterized in that** the compound which binds to β2GPI is selected from the group formed by an antibody directed against β2GPI, another protein and a biological or chemical compound which binds to β2GPI.

15. Process according to Claim 14, **characterized in that** the protein which recognizes β2GPI is the HIV₂ p26 ROD protein.

16. Process according to Claim 1, **characterized in that** the IC of the complex belongs to the group formed by HIV1, HIV2, HSV, HBV and particles or proteins of viral origin.

17. Process according to Claim 1, **characterized in that** the IC of the complex belongs to the group formed by bacteria, parasites, mycoplasmas and fungi.

18. Process according to Claim 7, **characterized in that** the IC of the β2GPI/IC complex, which is attached to the support, is separated and/or detected and/or quantified by infectivity, by a specific enzymic reaction, by a fluorescent or radioactively labelled tracer, by detecting nucleic acids by means of hybridization with a labelled probe or by means of a polymerase chain reaction (PCR) or else using (an) antibody(ies) which is/are specific for the IC.

19. Process according to one of Claims 1 to 18, **characterized in that** the form of β2GPI which is employed is β2'GPI, which is or is not in pure form.

20. Process according to one of Claims 1 to 19, **characterized in that** the β2GPI which is employed for assembling the complex with the IC('s) is labelled, with the complex being detected and/or quantified due to the said label.

21. Process according to Claim 20, **characterized in that** the β2GPI is labelled by means of an enzyme, a radioactive product or a fluorescent product.

22. Process according to Claim 6, **characterized in that** the β2GPI element is attached to the support before the β2GPI/IC complex is formed, **in that** the said complex is then formed, and **in that** the IC element of the complex is then detected and/or quantified.

23. Process according to one of Claims 1 to 22, **characterized in that** at least one of the elements of the complex is of animal origin or is produced by genetic and/or chemical engineering.
